# EUROPÄISCHE PATENTSCHRIFT

(11) **EP 0 691 322 B1**
(45) Veröffentlichungstag und Bekanntmachung des Hinweises auf die Patenterteilung: **22.04.1998**
(21) Anmeldenummer: 95109929.0
(22) Anmeldetag: 26.06.1995
(51) Int. Cl.: C07C 53/48, B01J 27/12, C07C 51/62

(54) **Verfahren zur Herstellung von Trifluoracetylfluorid**
Process for preparing trifluoroacétyl fluoride
Procédé de préparation de fluorure de trifluoroacétyle

(30) Priorität: 04.07.1994 DE 4423386
(43) Veröffentlichungstag der Anmeldung: 10.01.1996
(73) Patentinhaber: SOLVAY (Société Anonyme), B-1050 Bruxelles (BE)
(72) Erfinder: Ebmeyer, Frank, Dr., D-86153 Augsburg (DE); Metzenthin, Tobias, Dr., D-65929 Frankfurt (DE); Siegemund, Günter, Dr., D-65719 Hofheim (DE)
(74) Vertreter: Jacques, Philippe

(56) Entgegenhaltungen:
- EP-A- 0 130 532
- US-A- 3 859 424

## Beschreibung

Die Erfindung betrifft ein Verfahren zur Herstellung von Trifluoracetylfluorid durch Reaktion von Trichloracetylchlorid mit wasserfreiem Fluorwasserstoff in der Gasphase in Gegenwart eines chromhaltigen Fluorierungskatalysators.

Trifluoracetylfluorid wird als Ausgangsverbindung für die Herstellung zahlreicher Wirkstoffe auf dem Pharma- und Pflanzenschutzsektor verwendet oder zur Herstellung von Trifluoressigsäure eingesetzt.

Die Fluorierung von Trichloracetylchlorid durch Umsetzung mit Fluorwasserstoff in Gegenwart eines Fluorierungskatalysators, bestehend aus Chrom-(III)-oxid, in der Gasphase bei 250 - 325°C ist bereits bekannt. Dabei entsteht jedoch hauptsächlich Chlordifluoracetylfluorid (GB-PS 976 316), während bei einer vollständigen Fluorierung zu Trifluoracetylfluorid 26 % des Trichloracetylchlorids durch Zersetzung verloren gehen (US-PS 3 859 424).

Gemäß US-PS 3 787 489 wird die Fluorierung von Trichloracetylchlorid zu Trifluoracetylfluorid mit Hilfe eines Chrom-Katalysators in einem Reaktor vorgenommen, der drei unterschiedlich heiße Zonen enthält, um unerwünschte Zersetzungsreaktionen zu vermeiden. Dabei muß der Katalysator in den drei Heizzonen in verschiedenen, unterschiedlich beheizten Lagen aufgeschüttet werden, die ihrerseits durch Zwischenlagen aus Aluminiumoxid getrennt sind. Diese komplizierte Katalysatorschüttung ist technisch schwierig zu realisieren und erschwert außerdem Austausch und Reinigung des Katalysatorbetts.

Es wurde nun überraschend gefunden, daß bei der Verwendung eines bekannten, Chrom und Magnesium enthaltenden Fluorierungskatalysators ein Reaktorbetrieb mit drei unterschiedlich beheizten Zonen nicht erforderlich ist.

Gegenstand der Erfindung ist ein Verfahren zur Herstellung von Trifluoracetylfluorid durch Reaktion von Trichloracetylchlorid mit wasserfreiem Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500°C mit Fluorwasserstoff behandelt.

Der Katalysator und seine Vorbehandlung (Aktivierung) wird in der EP-B-0 130 532 (= US-PS 4 547 483) beschrieben, auf deren Lehre hiermit ausdrücklich Bezug genommen wird ("incorporation by reference"). Pro 1 Mol eines wasserlöslichen Chrom(III)-Salzes werden mindestens 1,5 Mol Magnesiumhydroxid oder Magnesiumoxid eingesetzt.

Das Verfahren wird im allgemeinen nach Art üblicher Gasreaktionen an Festbett-Katalysatoren durchgeführt, indem man das Gasgemisch aus Trichloracetylchlorid und Fluorwasserstoff durch ein heizbares Reaktionsrohr leitet, das mit dem anspruchsgemäßen Katalysator gefüllt ist.

Das Reaktionsrohr wird vorzugsweise vertikal aufgestellt und besteht aus einem gegen Fluorwasserstoff hinreichend resistenten Werkstoff, wie Nickel oder Stahl.

Zur Durchführung des Verfahrens werden Trichloracetylchlorid und Fluorwasserstoff im Gaszustand gemischt. Die Förderung der Edukte kann hierzu entweder (durch Erwärmen der Vorratsgefäße und Zuleitungen) gasförmig oder (durch Benutzung von Förderpumpen) flüssig erfolgen. Anschließend werden sie durch einen Vorerhitzer bzw. einen Verdampfer in den mit Katalysator gefüllten Reaktor geleitet. Die Ausgangssubstanzen werden vorteilhafterweise kontinuierlich zudosiert und in technischer Reinheit verwendet.

Bei Atmosphärendruck beträgt der Durchsatz an Trichloracetylchlorid zweckmäßigerweise 5 - 800 Gramm (ca. 0,03 bis 4,4 mol), insbesondere 30 - 300 Gramm (ca. 0,2 bis 2 mol), pro Liter Katalysator und Stunde. Bei höheren Drücken kann der Durchsatz entsprechend höher liegen.

Das Verfahren findet im allgemeinen bei Normaldruck oder geringem Überdruck statt, etwa 10⁻¹ bis 25 bar, vorzugsweise 1 bis 12 bar. Insbesondere zur Erzielung von höheren Raum-Zeit-Ausbeuten ist die Anwendung von Überdruck (2 bis 12 bar) vorzuziehen.

Das Molverhältnis von HF zu Trichloracetylchlorid beträgt im allgemeinen 4:1 bis 15:1, bevorzugt 5:1 bis 12:1.

Die Reaktion wird im allgemeinen bei Temperaturen von 200 bis 400°C, bevorzugt bei 250 bis 370°C durchgeführt, insbesondere bei 270 bis 340°C.

Die Verweilzeit des Gasgemisches im Reaktor beträgt im allgemeinen 1 bis 60 s, bevorzugt 5 bis 50 s.

Der Umsatz des eingesetzten Trichloracetylchlorids ist im allgemeinen vollständig.

Das bei der Umsetzung entstehende, Trifluoracetylfluorid, HCl und HF enthaltende Gas wird kondensiert und destillativ aufgearbeitet.

Die Vorteile des erfindungsgemäßen Verfahrens liegen in der einfachen Durchführung mit nur einer Reaktionszone, dem hohen Umsatz an Trichloracetylchlorid und der hohen Selektivität der Bildung von Trifluoracetylfluorid. Ein weiterer wichtiger Vorteil besteht darin, daß trotz der einfachen Durchführung mit nur einer Reaktionszone keine Zersetzungsreaktionen auftreten.

Das erfindungsgemäße Verfahren wird durch die nachfolgenden Beispiele näher erläutert. Der Umsatz erreichte in allen Beispielen 100 %. Die Prozentangaben in den Beispielen sind Gewichtsprozente, soweit nichts anderes gesagt wird.

### Versuchsbericht

### (Katalysatorherstellung gemäß EP-B-130 532 = US-PS 4 547 483)

200 g Cr(NO₃)₃ x 9 H₂O wurden in 1 l Wasser gelöst. Diese Lösung wurde zu einer Mischung von 500 g Magnesiumoxid und 240 g Graphit gegeben und die dabei entstehende pastöse Masse innig verknetet.

Anschließend wurde das pastöse Reaktionsprodukt zu Würfelförmlingen (0,5 cm Kantenlänge) granuliert und 16 Stunden bei 100°C getrocknet.

1 l (Schüttvolumen) der getrockneten Katalysatorkörper (= ca. 1000 g) wurden in einem Rohr aus Nickel oder VA-Stahl mit 5 cm lichter Weite und 100 cm Länge bei 200°C mit 15 mol Fluorwasserstoff behandelt. Die Dauer der Fluorwasserstoffbehandlung betrug ca. 6 Stunden. Dabei wurde das HF mit N₂ verdünnt. Der erhaltene Fluorierungskatalysator (Chrommagnesitkatalysator) wies einen Chromgehalt von 2,3 Gew.-% auf.

### Beispiel 1

Als Reaktor für die Umsetzung wurde ein Nickelrohr mit einem Innendurchmesser von 5 cm und einer Länge von 120 cm verwendet, welches von außen mit einer Ölheizung gleichmäßig beheizt wurde. Die Innentemperatur des Reaktors wurde mit Hilfe eines axialen Thermoelementes in einem Gehäuse bestimmt. Der vertikal installierte Reaktor wurde mit 1,0 kg (1,0 l) des gemäß Versuchsbericht hergestellten Chrommagnesitkatalysators beschickt.

Die Reaktionspartner wasserfreier Fluorwasserstoff (Reinheit > 99 %) und Trichloracetylchlorid (Reinheit > 99 %) wurden in einen vorgeschalteten, auf 125°C beheizten Verdampfer eindosiert. Hier erfolgte eine Durchmischung und Verdampfung der Reaktionspartner, die anschließend gasförmig in den gleichmäßig auf 340°C Innentemperatur beheizten und mit dem Katalysator gefüllten Reaktor geführt wurden. Bei einer Einspeisung von 65,1 g/h Fluorwasserstoff und 61,9 g/h Trichloracetylchlorid ergab die Analyse des aus dem Reaktor austretenden Gasgemisches:

| | |
|---|---|
| Trifluoracetylfluorid | 83 % |
| Difluormonochloracetylfluorid | 17 % |
| Fluordichloracetylfluorid | 0 % |
| Trichloracetylfluorid | 0 % |

Zersetzungsprodukte, wie z.B. fluorierte C1-Verbindungen, waren nicht entstanden.

### Beispiel 2

Es wurde wie in Beispiel 1 gearbeitet, außer daß 57 g/h Trichloracetylchlorid und 76 g/h Fluorwasserstoff eingespeist wurden. Die Analyse des aus dem Reaktor austretenden Gasgemisches ergab:

| | |
|---|---|
| Trifluoracetylfluorid | 86 % |
| Difluormonochloracetylfluorid | 14 % |
| Fluordichloracetylfluorid | 0 % |
| Trichloracetylfluorid | 0 % |

Zersetzungsprodukte waren nicht entstanden.

### Beispiel 3

Als Reaktor wurde ein elektrisch beheiztes Rohr aus V4A-Stahl mit einem Innendurchmesser von 5 cm und einer Länge von 70 cm verwendet, das mit 1,1 kg des gemäß Versuchsbericht hergestellten Chrommagnesitkatalysators gefüllt war. Der Wärmegradient wurde mit Hilfe eines axialen Thermoelementes bestimmt und betrug im vorliegenden Fall 270°C am kältesten Punkt und 340°C am heißesten Punkt der Katalysatorschüttung. Die Reaktionspartner wasserfreier Fluorwasserstoff und Trichloracetylchlorid wurden in eine auf 160°C beheizte Verdampferstrecke in Form eines 30 cm langen V4A-Rohres geleitet. Die durchmischten Komponenten gelangten anschließend in den elektrisch beheizten Reaktor, wo die Umsetzung zu Trifluoracetylfluorid erfolgte. Bei einer Dosierung von 28 g/h Trichloracetylchlorid und 28 g/h Fluorwasserstoff ergab die Analyse des aus dem Reaktor austretenden Gases:

| | |
|---|---|
| Trifluoracetylfluorid | 94,3 % |
| Difluormonochloracetylfluorid | 5,7 % |

Zersetzungsprodukte wie z.B. fluorierte C1-Verbindungen oder Derivate des bei einer Zersetzung von Trichloracetylchlorid entstehenden Phosgens bzw. Difluorphosgens waren nicht entstanden.

### Beispiel 4

In den im Beispiel 3 beschriebenen Reaktor dosierte man 45 g/h Trichloracetylchlorid und 46 g/h wasserfreien Fluorwasserstoff. Die beiden Reaktionspartner wurden durch die 250 - 260°C heiße Verdampferstrecke in den Reaktor geleitet, dessen Temperatur 270°C am kältesten Punkt und 340°C am heißesten Punkt betrug (Katalysatorschüttung 1,1 kg Chrommagnesit - Katalysator gemäß Versuchsbericht). Die regelmäßige Analyse der während einer Betriebszeit von 5 h aus der Reaktionszone austretenden Gase ergab die Zusammensetzung:

| | |
|---|---|
| Trifluoracetylfluorid | 86,0 % |
| Difluormonochloracetylfluorid | 14,0 % |

Andere Produkte waren nicht entstanden.

### Beispiel 5

In den im Beispiel 3 beschriebenen Reaktor dosierte man 40 g/h Fluorwasserstoff und 38 g/h Trichloracetylchlorid über die auf 217°C vorgeheizte Verdampferstrecke. Bei einer Temperaturverteilung zwischen einem Minimum von 260°C und einem Maximum von 390°C ergab die Umsetzung ein Produktgas der Zusammensetzung:

| | |
|---|---|
| Trifluoracetylfluorid | 95,4 % |
| Difluormonochloracetylfluorid | 3,4 % |
| Fluordichloracetylfluorid | 0,6 % |
| Difluorphosgen | 0,6 % |
| Trichloracetylfluorid | 0,0 % |

Weitere Nebenprodukte waren nicht entstanden.

## Patentansprüche

1. Verfahren zur Herstellung von Trifluoracetylfluorid durch Reaktion von Trichloracetylchlorid mit wasserfreiem Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500°C mit Fluorwasserstoff behandelt.

2. Verfahren nach Anspruch 1, dadurch gekennzeichnet, daß die Reaktion von Trichloracetylchlorid mit Fluorwasserstoff im Temperaturbereich von 200-400°C durchgeführt wird.

3. Verfahren nach Anspruch 1, dadurch gekennzeichnet, daß die Reaktion von Trichloracetylchlorid mit Fluorwasserstoff im Temperaturbereich von 250-370°C durchgeführt wird.

4. Verfahren nach einem der Ansprüche 1 bis 3, dadurch gekennzeichnet, daß die Reaktion von Trichloracetylchlorid mit Fluorwasserstoff unter einem Druck von 1-12 bar durchgeführt wird.

5. Verfahren nach einem der Ansprüche 1 bis 4, dadurch gekennzeichnet, daß die Reaktion von Trichloracetylchlorid mit Fluorwasserstoff bei einem Molverhältnis von 4 bis 15 Mol HF pro Mol Trichloracetylchlorid durchgeführt wird.

## Claims

1. A process for preparing trifluoroacetyl fluoride by reacting trichloroacetyl chloride with anhydrous hydrogen fluoride in the gas phase, which comprises using a chromium- and magnesium-containing catalyst which is obtainable by precipitating out chromium(III) hydroxide by reacting 1 mol of a water-soluble chromium(III) salt with at least 1.5 mol of magnesium hydroxide or magnesium oxide in the presence of water, converting the reaction mixture into a paste which contains chromium hydroxide and a magnesium salt and then drying the paste and treating it with hydrogen fluoride at temperatures of 20 to 500°C.

2. The process as claimed in claim 1, wherein the reaction of trichloroacetyl chloride with hydrogen fluoride is carried out in the temperature range 200-400°C.

3. The process as claimed in claim 1, wherein the reaction of trichloroacetyl chloride with hydrogen fluoride is carried out in the temperature range 250 - 370°C.

4. The process as claimed in one of claims 1 to 3, wherein the reaction of trichloroacetyl chloride with hydrogen fluoride is carried out at a pressure of 1 - 12 bar.

5. The process as claimed in one of claims 1 to 4, wherein the reaction of trichloroacetyl chloride with hydrogen fluoride is carried out at a molar ratio of 4 to 15 mol of HF per mole of trichloroacetyl chloride.

## Revendications

1. Procédé de préparation de fluorure de trifluoroacétyle par réaction de chlorure de trichloroacétyle avec le fluorure d'hydrogène exempt d'eau en phase gazeuse, caractérisé en ce qu'on utilise un catalyseur contenant du chrome et du magnésium, qui peut être obtenu par précipitation d'hydroxyde de chrome (III) en faisant réagir 1 mole d'un sel de chrome (III) soluble dans l'eau avec au moins 1,5 moles d'hydroxyde de magnésium ou d'oxyde de magnésium en présence d'eau, en transformant le mélange réactionnel en une pâte qui contient de l'hydroxyde de chrome et un sel de magnésium, en séchant ensuite cette pâte et en la traitant à des températures de 20 à 500 °C avec du fluorure d'hydrogène.

2. Procédé selon la revendication 1, caractérisé en ce que la réaction du chlorure de trichloroacétyle avec le fluorure d'hydrogène est effectuée à des températures dans la gamme de 200 à 400 °C.

3. Procédé selon la revendication 1, caractérisé en ce que la réaction du chlorure de trichloroacétyle avec le fluorure d'hydrogène est effectuée à des températures dans la gamme de 250 à 370 °C.

4. Procédé selon l'une des revendications 1 à 3, caractérisé en ce que la réaction du chlorure de trichloroacétyle avec le fluorure d'hydrogène est effectuée à une pression de 1 à 12 bars.

5. Procédé selon l'une des revendications 1 à 4, caractérisé en ce que la réaction du chlorure de trichloroacétyle avec le fluorure d'hydrogène est effectuée avec un rapport molaire de 4 à 15 moles de HF par mole de chlorure de trichloroacétyle.
